# EUROPÄISCHE PATENTSCHRIFT

(11) **EP 3 642 595 B1**
(45) Veröffentlichungstag und Bekanntmachung des Hinweises auf die Patenterteilung: **11.05.2022**
(21) Anmeldenummer: 18728139.9
(22) Anmeldetag: 30.05.2018
(51) Int. Cl.: G01N 15/06

(54) **RESISTIVER PARTIKELSENSOR**
RESISTIVE PARTICLE SENSOR
CAPTEUR DE PARTICULES RÉSISTIF

(30) Priorität: 23.06.2017 DE 102017210625
(43) Veröffentlichungstag der Anmeldung: 29.04.2020
(73) Patentinhaber: Robert Bosch GmbH, 70442 Stuttgart (DE)
(72) Erfinder: BAARS, Enno, 71229 Leonberg (DE); SCHILLING, Carolin Maria, 76669 Bad Schoenborn (DE); KLENK, Mathias, 74369 Loechgau (DE); HERWEG, Karola, 70469 Stuttgart (DE)
(86) Internationale Anmeldenummer: PCT/EP2018/064147
(87) Internationale Veröffentlichungsnummer: WO 2018/233994

(56) Entgegenhaltungen:
- DE-A1-102009 028 283
- US-A1- 2009 090 622

## Beschreibung

### Stand der Technik

Aus der US-20120119759 A1 ist bereits ein resistiver Partikelsensor zum Nachweis von Ruß im Abgas einer Brennkraftmaschine, mit einem Sensorelement mit zwei in einem einem Abgas aussetzbaren Bereich des Sensorelements beabstandet zueinander verlaufenden Leiterbahnen zum resistiven Nachweis einer Partikelmenge bekannt.

Dabei wird eine Partikelmenge mittels einer elektrischen Leitfähigkeit zwischen den Leiterbahnen sensiert.

Um die Abwesenheit von Ruß im Abgas von einer mangelnden Integrität des Partikelsensors unterscheiden zu können, wird gemäß dem Stand der Technik ein Widerstand vorgesehen, der die Leiterbahnen miteinander verbindet.

Nachteilig an dieser Anordnung ist, dass im Fall einer zwischen den Leiterbahnen vorliegenden Partikelmenge und einer daraus resultierenden leitenden Verbindung zwischen den Leiterbahnen die Feststellung der Integrität des Partikelsensors nicht mehr eindeutig möglich ist.

Aus der US 8,860,439 B2 ist ebenfalls ein resistiver Partikelsensor zum Nachweis von Ruß im Abgas einer Brennkraftmaschine, mit einem Sensorelement mit zwei in einem einem Abgas aussetzbaren Bereich des Sensorelements beabstandet zueinander verlaufenden Leiterbahnen zum resistiven Nachweis einer Partikelmenge bekannt. Die Leiterbahnen sind jeweils mit "plate conductors" verbunden und verlaufen in dem dem Abgas aussetzbaren Bereich des Sensorelements in einer geraden Linie beabstandet zueinander. Ein Wechselwirkungsbereich zwischen den Leiterbahnen ist daher vergleichsweise kurz. Die Sensitivität des Partikelsensors ist entsprechend gering.

Ein weiterer resistiver Partikelsensor ist aus der DE 101 33 384 A1 bekannt und weist kammförmige Leiterbahnen zum resistiven Nachweis einer Partikelmenge sowie einen in das Sensorelement integrierten mit den kammförmigen Leiterbahnen elektrisch verbundenen Plattenkondensator auf.

Weitere resistive Partikelsensoren sind bekannt aus US 2009/090622 A1 und DE 102009 028 283 A1.

### Offenbarung der Erfindung

Der erfindungsgemäße Partikelsensor weist Leiterbahnen auf, die in dem sensitiven Bereich in Mäandern parallel zueinander verlaufen. Ein Wechselwirkungsbereich zwischen den Leiterbahnen ist daher vergrößert.

Es ist erfindungsgemäß vorgesehen, dass die Leiterbahnen jeweils in einem Kapazitätselement enden und diese mit einem Kapazitätselement kapazitiv gekoppelt sind, welches elektrisch mit einer Widerstandsleiterbahn verbunden ist. Auf diese Weise ist die Integrität des Partikelsensors feststellbar.

Erfindungsgemäß ist vorgesehen, dass die Leiterbahnen verzweigungsfreie Leiterbahnen sind, die jeweils von einer außerhalb des dem Abgas aussetzbaren Bereichs angeordneten Kontaktfläche zur Kontaktierung des Sensorelements ausgehen, jeweils von der Kontaktfläche zu dem dem Abgas aussetzbaren Bereich des Sensorelements führen, dort in Mäandern verlaufen und nachfolgend zu den Kapazitätselementen führen und dass die Widerstandsleiterbahn von einer außerhalb des dem Abgas aussetzbaren Bereichs angeordneten Kontaktfläche zur Kontaktierung des Sensorelements ausgeht, und zu einer weiteren außerhalb des dem Abgas aussetzbaren Bereichs angeordneten Kontaktfläche zur Kontaktierung des Sensorelements führt und die Widerstandsleiterbahn ein Kapazitätselement aufweist und/oder mit einem Kapazitätselement über eine Zweigleiterbahn elektrisch verbunden ist.

Die Vorsehung der Kapazitätselemente außerhalb des dem Abgas aussetzbaren Bereichs verbessert die Lebensdauer des Partikelsensors und seine Messgenauigkeit über Lebensdauer.

Unter Leiterbahnen sind im Rahmen dieser Anmeldung insbesondere metallische Strukturen zu verstehen, die insbesondere lokal eine Längserstreckung und zu dieser und untereinander senkrecht eine Quererstreckung und eine Hocherstreckung aufweisen, wobei die Längserstreckung insbesondere wesentlich größer ist als die Quererstreckung und die Hocherstreckung.

Unter Leiterbahnen oder Leiterbahnabschnitten, die parallel zueinander verlaufen, sind im Rahmen dieser Anmeldung insbesondere Leiterbahnen oder Leiterbahnabschnitte zu verstehen, deren Längserstreckung lokal in die gleiche Richtung weist.

Unter Leiterbahnen, die in Mäandern verlaufen, sind im Rahmen dieser Anmeldung insbesondere Leiterbahnen zu verstehen, die mindestens zwei, bevorzugt mindestens drei oder mindestens vier, insbesondere parallel oder im Wesentlichen parallel zueinander verlaufende, Leiterbahnabschnitte aufweisen, wobei insbesondere zwischen benachbarten Leiterbahnabschnitten insbesondere Kehren der Leiterbahnen angeordnet sind, die insbesondere Kurven mit einem Winkel von 150° bis 210° sind.

Unter einem Kapazitätselement ist im Rahmen dieser Anmeldung insbesondere ein elektrischer Leiter zu verstehen, der zusammen mit einem weiteren Kapazitätselement und einem zwischen ihnen angeordneten elektrischen Isolator eine Kapazität ausbildet. Kapazitätselemente können beispielsweise flächig ausgebildet sein und eine Fläche von 25 bis 200 Quadratmillimeter aufweisen

Unter einer Kapazität ist im Rahmen dieser Anmeldung insbesondere eine Struktur aus zwei voneinander isolierten elektrischen Leitern (Kapazitätselementen) zu verstehen, die insbesondere von den oben erläuterten Leiterbahnen verschieden sind. Um eine Kapazität im Sinne der Erfindung zu qualifizieren, sind die zwei voneinander isolierten elektrischen Leiter insbesondere derart ausgebildet und voneinander beabstandet, dass sie bei einer Potentialdifferenz von einem Volt die Ladungsmenge 50-800 pC (Picocoulomb) zu speichern vermögen. Mit anderen Worten: Der Wert der Kapazität beträgt insbesondere 50 - 800 pF (Pikofarad).

Kapazitive Kopplungen, die geringer sind und/oder hinsichtlich der Funktionalitäten des Partikelsensors nicht bewusst herbeigeführt und/oder erwünscht sind, sondern im Rahmen kapazitiver Kopplungen sind, die zwangsläufig bei einem resistiven Partikelsensor auftreten, werde im Rahmen der Anmeldung nicht mit einer Kapazität im Sinne der Erfindung in Verbindung gebracht.

Es ist weiterhin besonders bevorzugt, wenn der Wert der Kapazität nicht weniger als 100 pF und/oder nicht mehr als 400 pF beträgt.

Unter einer Kontaktfläche zur Kontaktierung des Sensorelements sind im Rahmen dieser Anmeldung insbesondere Bereiche, beispielsweise Endbereiche, von Leiterbahnen zu verstehen, in denen eine Quererstreckung der Leiterbahn insbesondere vergrößert ist.

Es ist insbesondere vorgesehen, dass die Kapazität aus übereinander angeordneten metallischen vollflächigen schichtförmigen Kapazitätselementen und einer zwischen diesen angeordneten Isolationsschicht besteht. In diesem Fall ist der Wert der Kapazität besonders hoch.

Alternativ können die metallischen vollflächigen schichtförmigen Kapazitätselemente insbesondere durch metallische Gitter oder durch metallische Linienstrukturen ersetzt werden. In diesem Fall ist die Überdruckbarkeit der Kapazitätselemente besser.

### Kurze Beschreibung der Zeichnung

Figur 1 zeigt eine Übersicht über eines erfindungsgemäßen Partikelsensors
Figur 2 zeigt eine erste Ausführungsform in schematischer Form
Figur 3 zeigt eine zweite Ausführungsform in schematischer Form Ausführungsformen

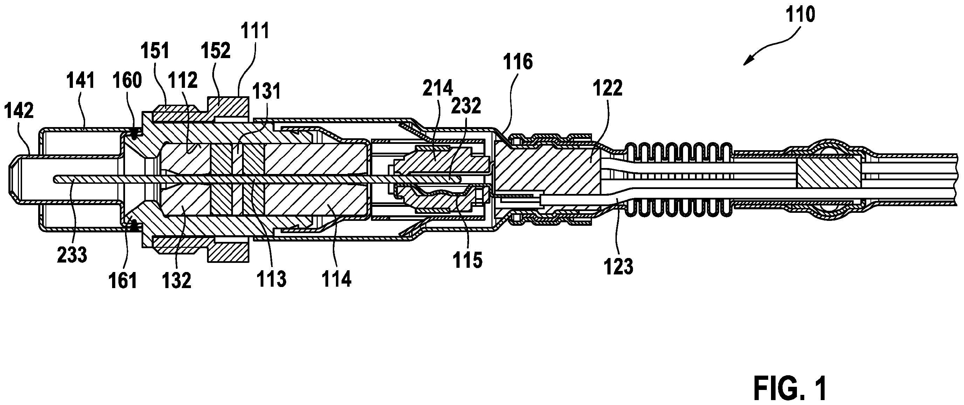

Figur 1 zeigt eine Übersicht über einen erfindungsgemäßen Partikelsensor 110 im Querschnitt entlang der Längsachse des Partikelsensors 110. Dieser Partikelsensor 110 weist ein metallisches Gehäuse 111 mit einer Durchgangsbohrung 112 auf, in der ein keramisches Sensorelement 113 durch eine Dichtpackung 131 sowie eine abgasseitige Isolationshülse 132 und eine kontaktseitigen Isolationshülse 114 festgelegt ist. Auf einen kontaktseitigen Endbereich des Sensorelements 232 sind beispielsweise 4 bis 6 Kontaktfedern 115 aufgeschoben, die wiederum in einem Kontakthalter 214 gehalten sind. Auf ihrer dem Abgas abgewandten Seite ist die Schutzhülse 116 durch eine Dichttülle 122 verschlossen, durch die mit den Kontaktfedern 115 elektrisch verbundene isolierte Leiter 123 hindurchgeführt sind.

Auf der dem Abgas zugewandten Seite des metallischen Gehäuses 111 sind zwei koaxiale Schutzrohre 141, 142 mittels einer gemeinsamen umlaufenden Schweißnaht 160 an einem abgasseitigen Kragen 161 des Gehäuses 111 fixiert. Die Schutzrohre 141, 142 weisen Öffnungen auf und decken einen abgasseitigen Endbereich des Sensorelements 233 ab. Dieser abgasseitige Endbereich des Sensorelements 233 ist somit ein dem Abgas aussetzbarer Bereich des Sensorelements 113. Der kontaktseitige Endbereich des Sensorelements 232 ist hingegen ein im Sinne der Erfindung nicht dem Abgas aussetzbarer Bereich.

Zur Montage in einem Abgassystem weist der Partikelsensor 110 ein Außengewinde 151 und ein Außensechskantprofil 152 auf.

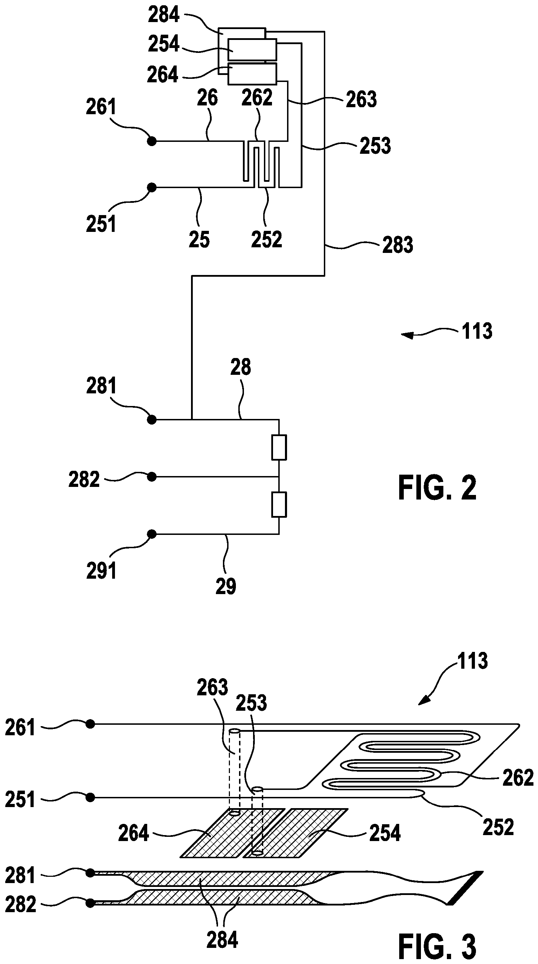

Figur 2 zeigt schematisch Elemente des Sensorelements 113 des resistiven Partikelsensors aus Figur 1. Dabei sind die Leiterbahnen 25, 26 verzweigungsfrei und gehen jeweils von einer außerhalb des dem Abgas aussetzbaren Bereichs angeordneten Kontaktfläche 251, 261 zur Kontaktierung des Sensorelements 113 aus. Von der Kontaktfläche 251, 261 führen die Leiterbahnen 25, 26 zu dem dem Abgas aussetzbaren Bereich des Sensorelements 113 wo sie in Mäandern 252, 262 parallel zueinander verlaufen. Nachfolgend verlaufen die Leiterbahnen 25, 26 über Zuleitungsabschnitte 253, 263 zu den Kapazitätselementen 254, 264, die im Beispiel flächig nebeneinander angeordnet sind.

Im Wesentlichen in einer Schichtebene unterhalb der Leiterbahnen 25, 26 verläuft eine Widerstandsleiterbahn 28, die im Beispiel ein Widerstandsheizer ist. Im Wesentlichen in einer nochmals unterhalb angeordneten Schichtebene verläuft eine weitere Widerstandsbahn 29, die im Beispiel eine Temperaturmess-Widerstandsbahn ist.

Die Widerstandsleiterbahn 28 und die weitere Widerstandsleiterbahn 29 weisen jeweils eine außerhalb des dem Abgas aussetzbaren Bereichs angeordnete Kontaktfläche 281, 291 zur Kontaktierung des Sensorelements 113 auf.

Die Widerstandsleiterbahn 28 und die weitere Widerstandsleiterbahn 29 führen zu einer weiteren außerhalb des dem Abgas aussetzbaren Bereichs angeordneten, im Beispiel gemeinsamen Kontaktfläche 282 zur Kontaktierung des Sensorelements 113. Die gemeinsame Kontaktfläche 282 ist beispielsweise mit einem Massepotential verbindbar.

Die Widerstandsleiterbahn 28 ist über eine Zweigleitung 283 mit dem Kapazitätselement 284 verbunden, das den Kapazitätselementen 254, 264 flächig und durch eine Isolationsschicht (nicht gezeichnet) getrennt außerhalb des dem Abgas ausgesetzten Bereichs des Sensorelements 113 gegenüberliegt.

Die beiden Kapazitätselemente 254, 264 sind vollflächig ausgebildet und nebeneinander angeordnet. Die beiden Kapazitätselemente 254, 264 und die Isolationsschicht bilden zusammen mit dem Kapazitätselement 284 eine Kapazität, deren Wert im Beispiel insgesamt 150pF (Pikofarad), 200pF oder 300pF betragen kann.

Eine zweite Ausführungsform ist in der Figur 3 gezeigt. Diese unterscheidet sich von der in der Figur 2 gezeigten Ausführungsform dadurch, dass das Kapazitätselement 284 der Widerstandsleiterbahn 28 nicht separat ausgebildet ist, sondern Teil der Widerstandsleiterbahn 28 ist. Im Beispiel wird das Kapazitätselement 284 durch verbreiterte Leiterbahnen der Widerstandleiterbahn 28 ausgebildet, die den Kapazitätselementen 254, 264 flächig und durch eine Isolationsschicht (nicht gezeichnet) getrennt gegenüberliegt. Die Kapazitätselemente 254, 264 sind im Beispiel in einer anderen Schichtebene angeordnet als die kontaktseitigen Teile der Leiterbahnen 25, 26. Zu diesem Zweck sind die Zuleitungsabschnitte 253, 254 als Durchkontaktierung durch eine Schicht des Sensorelements 113 ausgebildet.

Das Sensorelement 113 kann beispielsweise in der an sich bekannten Dickschicht-Technologie hergestellt werden.

## Patentansprüche

1. Resistiver Partikelsensor zum Nachweis von Ruß im Abgas einer Brennkraftmaschine, mit einem Sensorelement (113) mit zwei Leiterbahnen (25, 26) und einer Widerstandsleiterbahn (28), **dadurch gekennzeichnet, dass** die Leiterbahnen (25, 26) in einem dem Abgas aussetzbaren Bereich des Sensorelements (113) beabstandet in Mäandern (252, 262) parallel zueinander verlaufen und dass die zwei Leiterbahnen (25, 26) jeweils über Kapazitätselemente (254, 264, 284) mit der Widerstandsleiterbahn (28) kapazitiv verbunden sind und dass die Leiterbahnen (25, 26) verzweigungsfreie Leiterbahnen (25, 26) sind, die jeweils von einer außerhalb des dem Abgas aussetzbaren Bereichs angeordneten Kontaktfläche (251, 261) zur Kontaktierung des Sensorelements (113) ausgehen, jeweils von der Kontaktfläche (251, 261) zu dem dem Abgas aussetzbaren Bereich des Sensorelements (113) führen, dort in Mäandern (252, 262) verlaufen und nachfolgend zu den Kapazitätselementen (254, 264) führen und dass die Widerstandsleiterbahn (28) von einer außerhalb des dem Abgas aussetzbaren Bereichs angeordneten Kontaktfläche (281) zur Kontaktierung des Sensorelements (113) ausgeht und zu einer weiteren außerhalb des dem Abgas aussetzbaren Bereichs angeordneten Kontaktfläche (282) zur Kontaktierung des Sensorelements (113) führt und die Widerstandsleiterbahn (28) ein Kapazitätselement (284) aufweist und/oder mit einem Kapazitätselement (284) über eine Zweigleitung (283) elektrisch verbunden ist.

2. Resistiver Partikelsensor nach Anspruch 1, **dadurch gekennzeichnet, dass** die Kapazitätselemente (254, 264, 284) vollflächige Schichten sind, zwischen denen eine Isolationsschicht angeordnet ist.

3. Resistiver Partikelsensor nach Anspruch 1 **dadurch gekennzeichnet, dass** die Kapazitätselemente (254, 264, 284) metallische Gitter und/oder Linienstrukturen sind, zwischen denen eine Isolationsschicht angeordnet ist

4. Resistiver Partikelsensor nach einem der vorangehenden Ansprüche, **dadurch gekennzeichnet dass** der Wert der kapazitiven Verbindung 50 - 800 pF (Pikofarad) beträgt.

5. Resistiver Partikelsensor nach einem der vorangehenden Ansprüche, **dadurch gekennzeichnet dass** die Widerstandsleiterbahn (28) ein Widerstandsheizer und/oder eine Temperaturmess-Widerstandsleiterbahn ist.

## Claims

1. Resistive particle sensor for detecting soot in the exhaust gas of an internal combustion engine, comprising a sensor element (113) with two conductor tracks (25, 26) and a resistance conductor track (28), **characterized in that** the conductor tracks (25, 26) run parallel to one another at a distance in meanders (252, 262) in a region of the sensor element (113) that can be exposed to the exhaust gas, and **in that** the two conductor tracks (25, 26) are capacitively connected to the resistance conductor track (28) by means of respective capacitance elements (254, 264, 284), and **in that** the conductor tracks (25, 26) are branch-free conductor tracks (25, 26) which start from a respective contact area (251, 261), arranged outside the region that can be exposed to the exhaust gas, for making contact with the sensor element (113), lead from the respective contact area (251, 261) to the region of the sensor element (113) that can be exposed to the exhaust gas, there run in meanders (252, 262), and subsequently lead to the capacitance elements (254, 264), and **in that** the resistance conductor track (28) starts from a contact area (281), arranged outside the region that can be exposed to the exhaust gas, for making contact with the sensor element (113) and leads to a further contact area (282), arranged outside the region that can be exposed to the exhaust gas, for making contact with the sensor element (113) and the resistance conductor track (28) has a capacitance element (284) and/or is electrically connected to a capacitance element (284) via a branch line (283).

2. Resistive particle sensor according to Claim 1, **characterized in that** the capacitance elements (254, 264, 284) are full-surface layers between which an insulation layer is arranged.

3. Resistive particle sensor according to Claim 1, **characterized in that** the capacitance elements (254, 264, 284) are metal lattices and/or line structures between which an insulation layer is arranged.

4. Resistive particle sensor according to one of the preceding claims, **characterized in that** the value of the capacitive connection is 50 - 800 pF (picofarads).

5. Resistive particle sensor according to one of the preceding claims, **characterized in that** the resistance conductor track (28) is a resistance heater and/or a temperature-measuring resistance conductor track.

## Revendications

1. Capteur de particules résistif destiné à la détection de suie dans les gaz d'échappement d'un moteur à combustion interne, ledit capteur comprenant un élément capteur (113) pourvu de deux pistes conductrices (25, 26) et d'une piste conductrice résistive (28), **caractérisé en ce que** les pistes conductrices (25, 26) s'étendent à distance en méandres (252, 262) parallèlement les unes aux autres dans une zone de l'élément capteur (113) qui peut être exposée aux gaz d'échappement et **en ce que** les deux pistes conductrices (25, 26) sont chacune reliées de manière capacitive à la piste conductrice résistive (28) par le biais d'éléments capacitifs (254, 264, 284) et **en ce que** les pistes conductrices (25, 26) sont des pistes conductrices (25, 26) sans dérivation qui partent chacune d'une surface de contact (251, 261) disposée à l'extérieur de la zone pouvant être exposée aux gaz d'échappement et destinée à venir en contact avec l'élément capteur (113), qui vont chacune de la surface de contact (251, 261) à la zone de l'élément capteur (113) qui peut être exposée aux gaz d'échappement, qui s'étendent à cet endroit en méandres (252, 262), et qui aboutissent ensuite aux éléments capacitifs (254, 264) et **en ce que** la piste conductrice résistive (28) part d'une surface de contact (281) disposée à l'extérieur de la zone qui peut être exposée aux gaz d'échappement et destinée à venir en contact avec l'élément capteur (113) et aboutit à une autre surface de contact (282) disposée à l'extérieur de la zone qui peut être exposée aux gaz d'échappement et destinée à venir en contact avec l'élément capteur (113) et la piste conductrice résistive (28) comporte un élément capacitif (284) et/ou est reliée électriquement à un élément capacitif (284) par le biais d'une ligne de dérivation (283).

2. Capteur de particules résistif selon la revendication 1, **caractérisé en ce que** les éléments capacitifs (254, 264, 284) sont des couches pleine surface entre lesquelles est disposée une couche isolante.

3. Capteur de particules résistif selon la revendication 1, **caractérisé en ce que** les éléments capacitifs (254, 264, 284) sont des grilles métalliques et/ou des structures linéaires entre lesquelles est disposée une couche isolante.

4. Capteur de particules résistif selon l'une des revendications précédentes, **caractérisé en ce que** la valeur de la liaison capacitive est de 50 à 800 pF (picofarads).

5. Capteur de particules résistif selon l'une des revendications précédentes, **caractérisé en ce que** la piste conductrice résistive (28) est une résistance chauffante et/ou une piste conductrice résistive de mesure de température.
